# EUROPEAN PATENT SPECIFICATION

(11) **EP 3 175 894 B1**
(45) Date of publication and mention of the grant of the patent: **07.06.2023**
(21) Application number: 15827075.1
(22) Date of filing: 28.07.2015
(51) Int. Cl.: A63B 71/08, A63B 71/12, A63B 71/14, A61F 5/10, A61F 5/058, A61F 5/01

(54) **JOINT MOVEMENT SUPPORTING PROTECTOR**
SCHUTZ ZUR UNTERSTÜTZUNG EINER GELENKBEWEGUNG
DISPOSITIF DE PROTECTION DE SUPPORT DE MOUVEMENT ARTICULAIRE

(30) Priority: 29.07.2014 CN 201410365832
(43) Date of publication of application: 07.06.2017
(73) Proprietor: Yuan, Hongtao, Guangdong Province, 518103 (CN)
(72) Inventor: Yuan, Hongtao, Guangdong Province, 518103 (CN)
(74) Representative: Dehns
(86) International application number: PCT/CN2015/085291
(87) International publication number: WO 2016/015622

(56) References cited:
- CN-A- 104 117 198
- CN-U- 203 235 221
- CN-U- 204 121 708
- CN-Y- 2 162 267
- CN-Y- 2 741 614
- US-A- 3 938 207
- US-A- 5 255 391
- US-A1- 2005 054 487

## Description

### FIELD OF THE INVENTION

The present disclosure relates to the field of human body movement protection device, and particularly to a joint movement supporting protection device.

### BACKGROUND OF THE INVENTION

At present, joint protection devices are mainly divided into two types: elastic fiber fabric protection devices with rubber bands and plastic supporting protection devices. Common elastic fiber fabric protection devices with rubber bands include ankle protection devices, wrist protection devices, knee protection devices, waist protection devices, finger protection devices and so on. Their principles are that material elasticity is utilized to increase joint stability so as to buffer and prevent exterior injury and excessive deformation to some extent. However, since the elastic fiber fabric protection devices with rubber bands are simplex in material and structure, so the physical performance in all directions are identical, and thus they are limited in protection of joints under the requirement of giving consideration to joint movement functions. The reason is very simple: binding strength needs to be enhanced to increase protection to joints, which may have a negative effect on joint movement; instead, protection to the joints may be reduced if the binding strength is reduced to take the joint movement into account. Common plastic supporting protection devices include knee protection devices in skating and skiing sports, and finger joint supporting protection devices in football goalkeeper gloves. This type of protection devices make up for some deficiencies of the former type of protection devices. However, at present, this type of protection devices are larger in structural volume, unsuited to bodies, uncomfortable for wearing, complex in structure, and thus relatively high in cost.

To a basketball fan, finger injury is not fatal as knee injury or ankle injury. However, fingers are slender and vulnerable, unable to bear great strength, and are in contact with a basketball momently. Therefore, fingers are more susceptible to get injured. Once the fingers are injured, a seriously negative effect may be undoubtedly caused on the basketball sport. Injured fingers may be more vulnerable, and thus may be easily susceptible to repeated injury, which causes the injured fingers hardly be restored for a long time. In the field of basketball sports, a basketball finger protection device that can provide effective protection to fingers has been always expected to appear. However, at present, there is only one type of finger protection device in the market, namely, an elastic barrel-shaped finger protection device with a rubber band. However, this type of finger protection device is not comfortable and is limited in protection of fingers.

CN 2 741 614 Y describes a finger joint motion support brace, which is composed of a front end piece, a rear end piece and a plurality of joint pieces disposed between the front end piece and the rear end piece, and is shaped into an arc shape in a normal state. It is elastically assembled in various ball receiving devices, and is characterized in that the front end piece portion, the rear end piece portion and the plurality of joint pieces disposed between the front end piece portion and the rear end piece portion are integrally formed by a single mold. Multiple outer joint pieces are formed, wherein one is furthermore a flexible connecting piece which is integrally formed between adjacent joint pieces and between the joint piece and the front end piece part and the rear end piece part.

Therefore, it is still a problem to be solved in the art to develop a finger protection device that can provide effective protection to fingers and is comfortable. US 3,938,207 discloses a swimmer's glove having joints only bendable in the closing direction of the hand.

### SUMMARY OF THE INVENTION

A technical problem solved by the present disclosure resides in overcoming the above defects and providing a joint movement supporting protection device which is light and thin in volume, simple in structure, can be firmly attached to a joint part, does not affect normal joint movement, and is low in cost.

Embodiments of the invention provide a joint movement supporting protection device, including a joint protection body matched with a joint in shape, and a fixing device in connection or contact with the joint protection body and tightly attaching the joint protection body to a joint of a human body, wherein: a cross section of the joint protection body (1) is arc-like, a plurality of upper notches (11) are formed in a portion, close to a joint movement area, of the joint protection body (1); the upper notches (11) divide the joint protection body (1) into a plurality of joint pieces (12) with some connecting parts of the joint protection body (1) that connect adjacent joint pieces (12) together, and adjacent joint pieces (12) are capable of rotating around the connecting parts as fulcrums (13); when the joint pieces (12) rotate to a limiting position around the fulcrums (13), the joint pieces (12) do not rotate any more since adjacent joint pieces (12) contact to each other.

Further, lower notches are further formed in positions, extending downwards from a bottom side of the upper notches, the lower notches correspond to the upper notches one by one, a depth of the lower notch is less than a depth of the upper notch, a junction between the upper notch and the lower notch constitutes a rotation fulcrum between two adjacent joint pieces.

Further, a ratio of the depth of the lower notch to the depth of the upper notch is 1:2~1:8.

Further, edges of the upper notch or the lower notch and the joint piece are rounded off.

Preferably, bottom width of the upper notch is larger than top width thereof.

Preferably, heights of a plurality of the joint pieces increase progressively, and each joint piece extends outwardly and covers over top of the upper notch.

Preferably, the fixing device includes a plurality of strip-shaped fixing belts, an end of the fixing belt and a side of the joint protection body are integrally molded, a tail end of the fixing belt is provided with at least one buckle, the joint protection body is provided with a through hole matched with the buckle; after wrapping the joint, the fixing belt is fastened to the joint protection body by fastening the buckle to the through hole.

Further, a tail end of the fixing belt may be further provided with at least one saw-toothed barb, and the joint protection body is provided with a corresponding through hole or semi-through hole matched with the saw-toothed barb.

Preferably, the fixing device includes fixing rings arranged on two ends of the joint protection body; the fixing rings are integrally molded with the joint protection body.

Further, the fixing device is a closed ring or a split ring.

Preferably, the fixing device is an elastic fabric fixed to the joint protection body, or the fixing device is an independent fixing belt, or the fixing device is a shoe.

Preferably, the joint protection body is fixed to a shoe.

Based on the above technical features, the joint movement supporting protection device proposed in the embodiments of the invention can be firmly attached to a joint part, does not affect the normal joint movement, and effectively protect joints; moreover, it is simple in structure and low in cost, and can be efficiently and conveniently worn and detached.

### BRIEF DESCRIPTION OF THE DRAWINGS

FIG. 1 is a front perspective view of a joint movement supporting protection device according to the present disclosure;
FIG. 2 is a side perspective view of the joint movement supporting protection device according to the present disclosure;
FIG. 3 is a side sectional view of the joint movement supporting protection device according to the present disclosure;
FIG. 4 is a use state diagram I of the joint movement supporting protection device according to the present disclosure;
FIG. 5 is a use state diagram II of the joint movement supporting protection device according to the present disclosure;
FIG. 6 is a sectional view of a fastened fixing belt of the joint movement supporting protection device according to the present disclosure;
FIG. 7 is a side view of the joint movement supporting protection device according to the present disclosure; and
FIG. 8 is a partial sectional view of the joint movement supporting protection device according to the present disclosure; and
FIG. 9 is a side perspective view of a joint movement supporting protection device according to another embodiment of the present disclosure; and
FIG. 10 is a side view of a joint movement supporting protection device according to another embodiment of the present disclosure; and
FIG. 11 is a front perspective view of a joint movement supporting protection device according to another embodiment of the present disclosure; and
FIG. 12 is a side perspective view of a joint movement supporting protection device according to another embodiment of the present disclosure; and
FIG. 13 is a front perspective view of a joint movement supporting protection device according to another embodiment of the present disclosure; and
FIG. 14 is a front perspective view of a joint movement supporting protection device according to another embodiment of the present disclosure; and
FIG. 15 is a use diagram I of a joint movement supporting protection device according to another embodiment of the present disclosure;
FIG. 16 is a use diagram II of a joint movement supporting protection device according to another embodiment of the present disclosure.

### DETAILED DESCRIPTION OF THE EMBODIMENTS

In order to make the aims, technical solutions and advantages of the present invention clear, the technical solution of the present disclosure is described in detail in the following with reference to specific embodiments and drawings of specification. It is understood that the following specific embodiments are merely illustrative and not for limiting.

The implementation of the present disclosure is described in detail in the following with reference to specific embodiments.

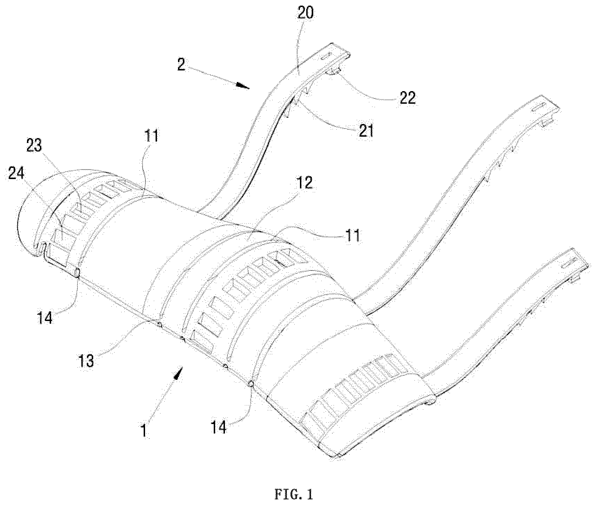

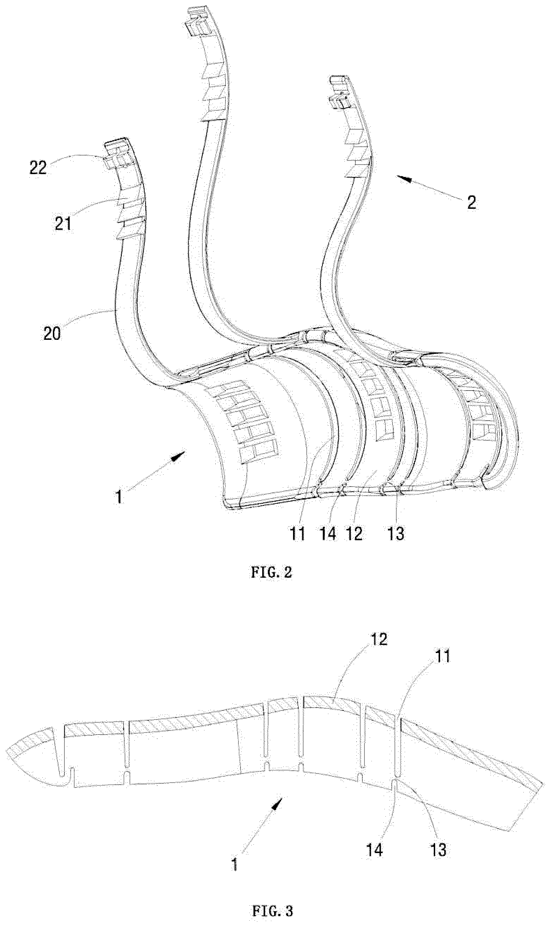

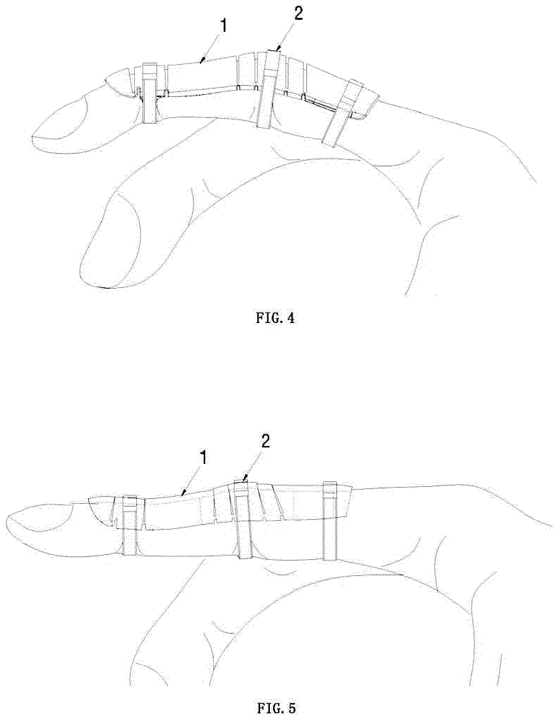

As shown in FIG. 1 to FIG. 8, embodiments of the present disclosure provide a joint movement supporting protection device, including a joint protection body 1, whose cross section is arc-like, matched with a joint in shape, size and length, and a fixing device 2 in connection or contact with the joint protection body and tightly attaching the joint protection body to a joint of a human body. Specifically, a plurality of upper notches 11 extending downwards from top but not penetrating through bottom are formed in a portion, close to a joint movement area, of the joint protection body 11, thus the joint protection body 1 is divided, by the upper notches 11, into a plurality of joint pieces 12, i.e. a plurality of joint pieces 12 are formed among a plurality of upper notches 11; at the same time, a junction between the upper notch 11 and a lower edge of the joint protection body 1 constitutes a rotation fulcrum 13 between adjacent two joint pieces 12; the adjacent joint pieces 12 can rotate around the rotation fulcrum 13, and when people wear the joint movement supporting protection device and keep the fingers straight, the adjacent joint pieces 12 do not rotate any more after they come into contact with each other. In this way, the joint is prevented from overbending toward a direction to back of a hand, thereby achieving the aim of protecting a finger. Regarding to finger joints, most importantly, the finger needs to be prevented from overbending toward the back of the finger, which is a primary cause to injury to the finger joints. The case of the above embodiment may be adopted when restricting the finger joints from bending toward a direction of a palm of the hand is left out of account.

In an embodiment of the present disclosure, lower notches 14 are further formed in positions, extending downwards from a bottom side, of corresponding positions of the upper notches 11, the lower notches 14 correspond to the upper notches 11, a depth of the lower notch 14 is less than a depth of the upper notch 11, specifically, an optional range is as below: a ratio of the depth of the lower notch 14 to the depth of the upper notch 11 is 1:2-1:8, thus the joint protection body 1 is divided, by the upper notches 11 and the lower notches 14, into a plurality of joint pieces 12, and a junction between the upper notch 11 and the lower notch 14 constitutes a rotation fulcrum 13 between adjacent joint pieces. Two adjacent joint pieces of the upper notch 11 just come into contact with each other when the joint is straightened, at the moment the adjacent joint pieces 12 do not rotate anymore because of a rigid support action of the joint protection body, so that the joint is prevented from overbending toward the direction of the back of the hand, thereby achieving the aim of protecting the finger. When the joint bends toward the direction of the palm of the hand to a certain extent, two adjacent joint pieces 12 of the lower notch 14 just come into contact with each other, at the moment the adjacent joint pieces 12 of the lower notch 14 do not rotate anymore because of a rigid support action of the joint protection body, so that the joint is prevented from overbending toward the direction of the palm of the hand, thereby achieving the aim of protecting the finger.

In an embodiment of the present disclosure, edges of the upper notch 11 or the lower notch 14 and the joint piece 12 are rounded off. Here, edges of the upper notch 11 or the lower notch 14 and the joint piece 12 are rounded off to improve fitness to the human body and performance.

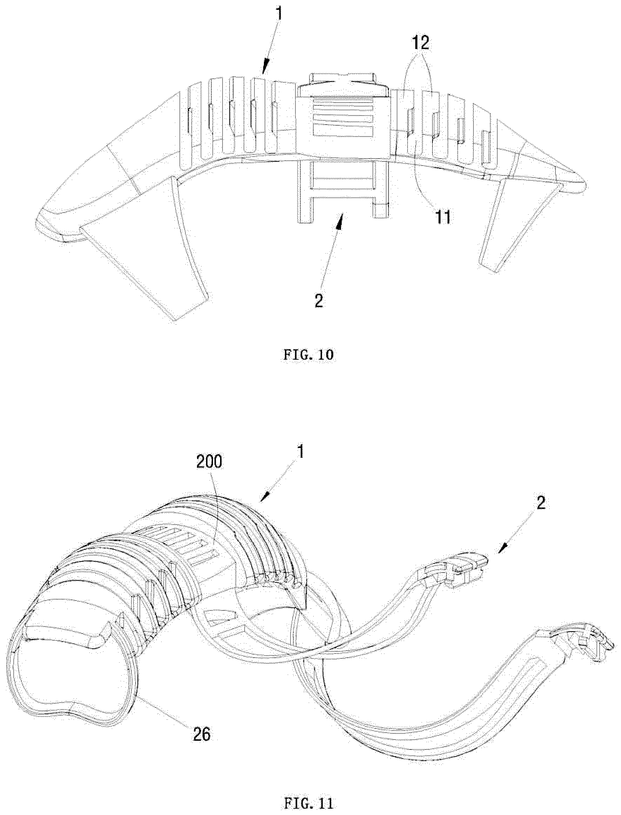

In another embodiment of the present disclosure, as shown in Figure 10, bottom width of the upper notch 11 is larger than top width thereof, so that rotating part connecting the adjacent two joint pieces 12 is stronger and not easy to break, thus the overall resilience of the joint movement supporting protection device is improved. Of course, according to actual demands, in other embodiment, it is possible to take some other means to improve the overall resilience of the joint movement supporting protection device.

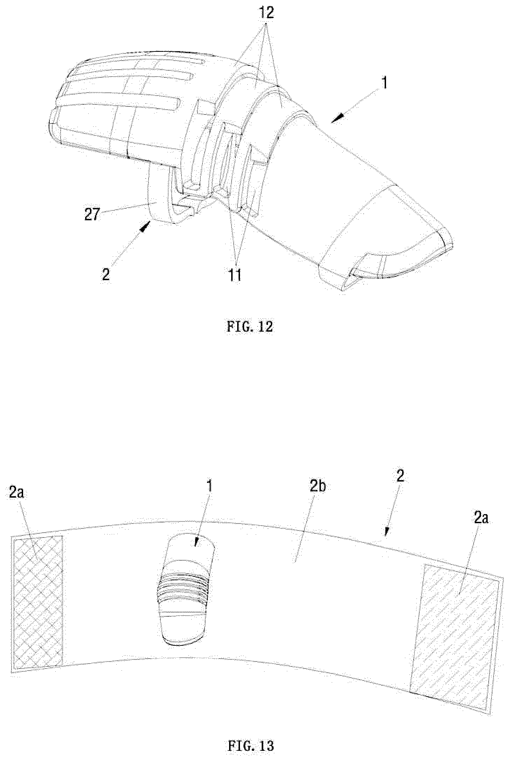

In an embodiment of the present disclosure, as shown in figure 12, heights of a plurality of the joint pieces 12 increase progressively, and each joint piece 12 extends outwardly and covers over top of the upper notch 11, thus, when finger joints are bend to some extent in a direction towards palm, the adjacent two joint pieces 12 rotate reversely around the rotation fulcrum 13, then, top of the upper notch 11 opens, since edge of each joint piece 12 extends outwards and cover over top of the upper notch 11, top of the upper notch 11 will not expose entirely, preventing injury from external objects to fingers or invasion of external objects, thus the overall safety of the joint movement supporting protection device is improved.

In an embodiment of the present disclosure, a plurality of strip-shaped fixing belts 20 are adopted for the fixing device 2, an end of the fixing belt 20 and a side of the joint protection body 1 are integrally molded, a tail end of the fixing belt 20 is provided with at least one buckle 22, correspondingly, the joint protection body 1 is provided with a plurality of through holes 23 matched with the buckle 22; after wrapping the joint, the fixing belt 2 is fastened to the joint protection body 1 by fastening the buckle 22 to the through holes 23 disposed on the joint protection body 1 so that the joint movement supporting protection device is tightly fastened to the joint. Of course, according to actual demands, in other embodiment, the fixing device 2 can have some other structure, and it is not limited.

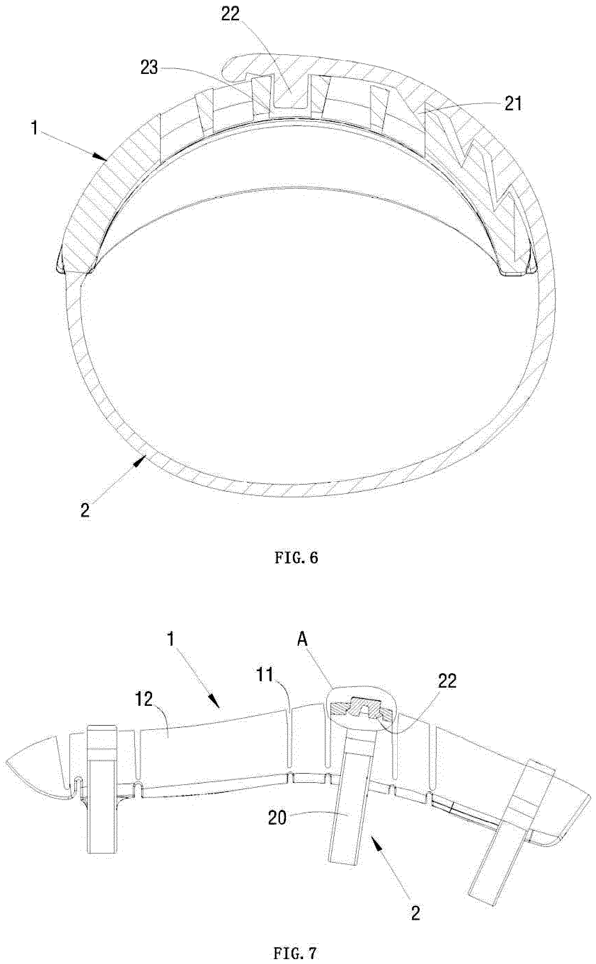

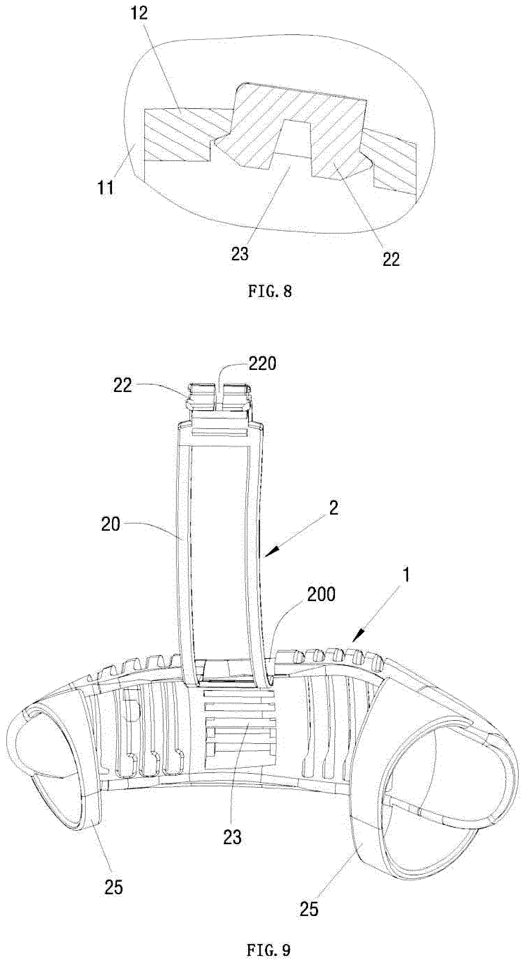

In an embodiment of the present disclosure, an end of the fixing belt 2 may be further provided with at least one saw-toothed barb 21, correspondingly, the joint protection body 1 is provided with a plurality of through holes 23 or semi-through holes 24 matched with the saw-toothed barb 21, so that the saw-toothed barb 21 is fastened to the through hole 23 or semi-through hole 24. FIG. 7 and FIG. 8 display a detail state that the buckle 22 is fastened to the through hole 23. The saw-toothed barb 21 and the buckle 22 cooperate with different through holes 23 or semi-through holes 24 so that a degree of tightness of the fixing belt 2 can be adjusted. In this way, the same joint protection body can fit to fingers of different sizes to a certain extent.

In other embodiments of the present disclosure, according to requirements for fixation of protection devices in different fields, a suitable structural style adopting other types may be selected for the fixing device 2. For example, a traditional Velcro tape is adopted for fixation, the joint protection body 1 may be inlaid and fixed into a glove or an elastic fabric, or can be fixed by an independent fixing belt, which can adopt a material in a proper form and match with a buckle having a proper structure to fasten the joint protection body 1. In this way, the joint protection body 1 is fastened to the joint of the human body.

As shown in Figure 9, in another embodiment of the present disclosure, the above fixing device 2 may include the above fixing belt 20 and a fixing ring 25 arranged on two ends of the joint protection body 1. The fixing ring 25 is integrally molded with the joint protection body 1 to form a soft plastic annular structure, so that the fixing ring 25 and the joint protection body 1 form one piece. At the same time, the joint protection body 1 is provided with a containing slot 200 to accommodate the fixing belt 20 on the back, i.e. when the fixing belt 20 surrounds the back of the joint protection body 1, the outer end portion of the fixing belt 20 is accommodated in the containing slot 200, so that the height of the fixing belt 20 is flush with the back of the joint protection body 1, avoiding being unaesthetic and scratch; furthermore, an opening 220 is provided at the middle of the buckle 22, thanks to the opening 220, the buckle 22 can access to the above through hole 23 more easily, improving convenience to take on and off.

Alternatively, as shown in Figure 11, the fixing device 2 further includes a closed annular fixing structure formed from a single material by single shot shaping technique, i.e. a closed ring 26 is formed between the fixing device 2 and the joint protection body 1, and as the material of the above joint protection body 1, the fixing device 2 is formed by single shot shaping technique. Here, the closed ring 26 is set to be corrugation, so that it can adapt to joint of different sizes, broadening the application of the joint movement supporting protection device and meeting people's different demands. At the same time, the joint protection body 1 is also provided with a containing slot 200 to accommodate the fixing belt 20 on the back, avoiding being unaesthetic and scratch.

Alternatively, as shown in Figure 12, the fixing device 2 further includes a split annular fixing structure formed from a single material by single shot shaping technique, i.e. a split ring 27 is formed between the fixing device 2 and the joint protection body 1, and as the material of the above joint protection body 1, the fixing device 2 is formed by single shot shaping technique. Thus, it can adapt to joint of different sizes, broadening the application of the joint movement supporting protection device and meeting people's different demands.

Alternatively, the joint protection body 1 can be fixed on joint by the following means: the joint protection body 1 matched with the shape and structure of joint to be protected may be inlaid or fixed into a fabric, and then the fabric is wrapped around the joint and fixed by Velcro. Specifically, as shown in figure 13, for example, in order to protect knee-joint, the fixing device 2 includes Velcro 2a and fabric 2b, and the joint protection body 1 is disposed on fabric 2b, when in use, the joint protection body 1 is positioned on the knee-joint, then the fabric 2b is wrapped around the leg, and at last, Velcro 2a on two ends of the fabric 2b are engaged.

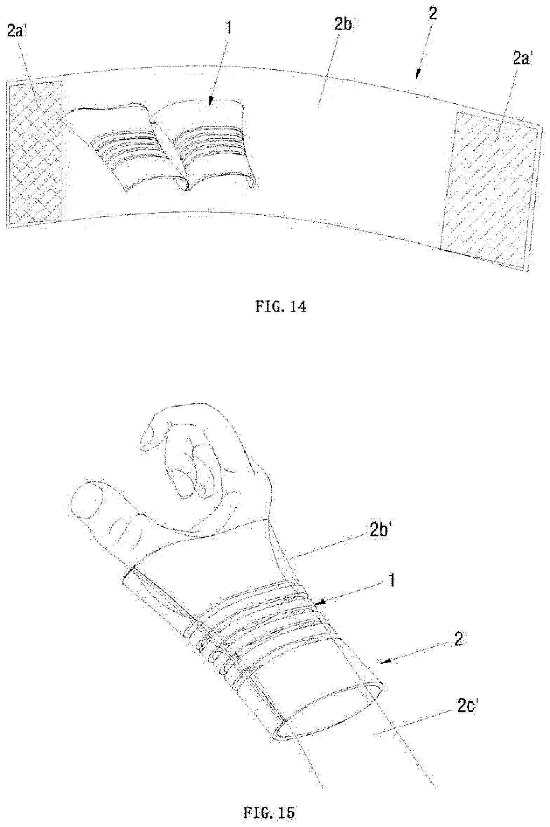

Furthermore, if people need to restrict movement of joint in multiple directions, a plurality of joint protection bodies 1 can be used, i.e. the joint protection body 1 matched with the shape and structure of joint to be protected may be inlaid or fixed into a fabric with certain shape and size, and then the fabric is wrapped around the joint and fixed by Velcro. Specifically, as shown in figures 14-15, for example, in order to protect wrist joint, the fixing device 2 includes Velcro 2a' and fabric 2b', and two joint protection bodies 1 is disposed on fabric 2b', when in use, the two joint protection bodies 1 are positioned on the wrist joint of an arm 2c' oppositely, then the fabric 2b' is wrapped around the arm 2c', and at last, Velcro 2a' on two ends of the fabric 2b' are engaged. The protection device can applied to some other body parts, such as elbow, ankle, waist, wherein the structures and principles are the same, but different shapes should be designed for different body parts to be protected.

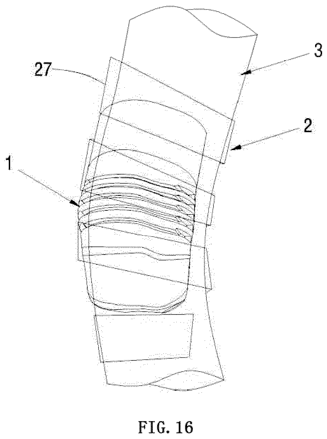

Alternatively, the above joint protection body 1 can be used in combination with self-adhesive non-woven fabrics or various tape or gummed paper or some other known fixing means, directly applied to protecting devices of exercise or medical rehabilitation devices. For example, in order to protect knee-joint, see Figure 16, the fixing device 2 includes self-adhesive non-woven fabric 27 (or tape or gummed paper), and the joint protection body 1 is positioned tightly on knee-joint 3, then joint protection body 1 is fixed on the knee-joint 3 through self-adhesive non-woven fabric.

In other embodiments of the present disclosure, the above joint protection body 1 can also be inlaid into shoes in a position where some parts need to be protected, preventing ankle from being sprained without obstructing movement of feet; alternatively, the above joint protection body 1 can also be inlaid into sporting gloves (such as goalkeeper's gloves) as a reinforce, to withstand the impact when hands catch a football, to prevent injuries to the fingers; alternatively, the above joint protection body 1 can also be applied as an internal layer of surface decorative parts of artificial limbs, as a support of the decorative parts; through the joint protection body 1, the structures of the artificial limbs can be simplified, making it more aesthetic, while keeping it flexible.

Application of the patent structure to finger protection in basketball is one of embodiments most fully reflecting advantages of the patent structure. Therefore, this patent is expounded mainly by means of an embodiment in which the joint movement supporting protection device is applied to finger protection in basketball. To applications in other aspects such as a football goalkeeper glove support, finger protection for playing musical instruments, finger protection for vegetable cutting, and even protection of wrists, ankles and knees or the like in ball games and outdoor sports, their structures and principles are roughly identical, merely shapes, sizes and so on are different. Therefore, these applications all fall within the scope of protection of this patent.

Based on the above technical features, the joint movement supporting protection device proposed in the embodiments of the invention can be firmly attached to a joint part, does not affect the normal joint movement, and effectively protect joints; moreover, it is simple in structure and low in cost, and can be efficiently and conveniently worn and detached.

Preferable embodiments of the invention are specifically described above, but the invention is not limited to the embodiments, the skilled familiar to the art can make various equivalent transformations and substitutions without departing from the invention as defined by claims.

## Claims

1. A joint movement supporting protection device, comprising
a joint protection body (1) matched with a joint in shape, and a fixing device (2) in connection or contact with the joint protection body (1) and tightly attaching the joint protection body (1) to a joint of a human body, **characterised in that**: a cross section of the joint protection body (1) is arc-like, a plurality of upper notches (11) are formed in a portion, close to a joint movement area, of the joint protection body (1); the upper notches (11) divide the joint protection body (1) into a plurality of joint pieces (12) with some connecting parts of the joint protection body (1) that connect adjacent joint pieces (12) together, and adjacent joint pieces (12) are capable of rotating around the connecting parts as fulcrums (13); when the joint pieces (12) rotate to a limiting position around the fulcrums (13), the joint pieces (12) do not rotate any more since adjacent joint pieces (12) contact to each other.

2. The joint movement supporting protection device according to claim 1, **characterized in that** corresponding lower notches (14) are further formed in positions, extending downwards from a bottom side, of corresponding positions of the upper notches (11), the lower notches (14) correspond to the upper notches (11) one by one, a depth of the lower notch (14) is less than a depth of the upper notch (11), and a junction between the upper notch (11) and the lower notch (14) constitutes a rotation fulcrum (13) between adjacent joint pieces (12).

3. The joint movement supporting protection device according to claim 2, **characterized in that** a ratio of the depth of the lower notch (14) to the depth of the upper notch (11) is 1:2~1:8.

4. The joint movement supporting protection device according to claim 2, **characterized in that** edges of the upper notch (11) or the lower notch (14) and the joint piece (12) are rounded off.

5. The joint movement supporting protection device according to claim 1, **characterized in that** bottom width of the upper notch (11) is larger than top width thereof.

6. The joint movement supporting protection device according to claim 1, **characterized in that** heights of a plurality of the joint pieces (12) increase progressively, and each joint piece (12) extends outwardly and covers over top of the upper notch (11).

7. The joint movement supporting protection device according to claim 1, **characterized in that** a plurality of strip- shaped fixing belts (20) are adopted for the fixing device (2), an end of the fixing belt (20) and a side of the joint protection body (1) are integrally molded, a tail end of the fixing belt (20) is provided with at least one buckle (22), the joint protection body (1) is provided with a corresponding through hole (23) matched with the buckle (22); after wrapping the joint, the fixing belt (20) is fastened to the joint protection body (1) by fastening the buckle (22) to the through hole (23).

8. The joint movement supporting protection device according to claim 7, **characterized in that** an end of the fixing belt (20) is further provided with at least one saw-toothed barb (21), and the joint protection body (1) is further provided with a corresponding through hole (23) or semi-through hole (24) matched with the saw-toothed barb (21).

9. The joint movement supporting protection device according to claim 1, **characterized in that** the fixing device (2) includes fixing rings (25) arranged on two ends of the joint protection body (1), the fixing rings (25) are integrally molded with the joint protection body (1).

10. The joint movement supporting protection device according to claim 1, **characterized in that** the fixing device (2) is a closed ring (26) or a split ring (27).

11. The joint movement supporting protection device according to claim 1, **characterized in that** the fixing device (2) is an elastic fabric fixed to the joint protection body (1), or the fixing device (2) is an independent fixing belt, or the fixing device (2) is a shoe.

12. The joint movement supporting protection device according to claim 1, **characterized in that** the joint protection body (1) is fixed to a shoe.

## Patentansprüche

1. Gelenkbewegungs-Stützschutzvorrichtung, umfassend
einen Gelenkschutzkörper (1), dessen Form zu einem Gelenk passt, und eine Befestigungsvorrichtung (2) in Verbindung oder Berührung mit dem Gelenkschutzkörper (1) und die den Gelenkschutzkörper (1) eng an einem Gelenk eines menschlichen Körpers anbringt, **dadurch gekennzeichnet, dass**: ein Querschnitt des Gelenkschutzkörpers (1) bogenförmig ist, eine Vielzahl oberer Kerben (11) in einem Teil nahe einem Gelenkbewegungsbereich des Gelenkschutzkörpers (1) gebildet ist; die oberen Kerben (11) den Gelenkschutzkörper (1) in eine Vielzahl von Gelenkstücken (12) aufteilen, mit einigen Verbindungsteilen des Gelenkschutzkörpers (1), die aneinandergrenzende Gelenkstücke (12) miteinander verbinden, und angrenzende Gelenkstücke (12) in der Lage sind, um die Verbindungsteile als Hebelpunkte (13) zu drehen; wenn die Gelenkstücke (12) zu einer Begrenzungsposition um die Hebelpunkte (13) drehen, die Gelenkstücke (12) nicht mehr drehen, da die aneinandergrenzenden Gelenkstücke (12) einander berühren.

2. Gelenkbewegungs-Stützschutzvorrichtung nach Anspruch 1, **dadurch gekennzeichnet, dass** entsprechende untere Kerben (14) weiter in Positionen gebildet sind, die sich von einer Unterseite entsprechender Positionen der oberen Kerben (11) nach unten erstrecken, die unteren Kerben (14) den oberen Kerben (11) einzeln entsprechen, eine Tiefe der unteren Kerbe (14) kleiner als eine Tiefe der oberen Kerbe (11) ist, und eine Verbindungsstelle zwischen der oberen Kerbe (11) und der unteren Kerbe (14) einen Drehpunkt (13) zwischen aneinandergrenzenden Gelenkstücken (12) bildet.

3. Gelenkbewegungs-Stützschutzvorrichtung nach Anspruch 2, **dadurch gekennzeichnet, dass** das Verhältnis der Tiefe der unteren Kerbe (14) zu der Tiefe der oberen Kerbe (11) 1:2~1:8 beträgt.

4. Gelenkbewegungs-Stützschutzvorrichtung nach Anspruch 2, **dadurch gekennzeichnet, dass** die Kanten der oberen Kerbe (11) oder der unteren Kerbe (14) und das Gelenkstück (12) gerundet sind.

5. Gelenkbewegungs-Stützschutzvorrichtung nach Anspruch 1, **dadurch gekennzeichnet, dass** die Grundbreite der oberen Kerbe (11) breiter als die Oberseitenbreite davon ist.

6. Gelenkbewegungs-Stützschutzvorrichtung nach Anspruch 1, **dadurch gekennzeichnet, dass** Höhen einer Vielzahl der Gelenkstücke (12) allmählich zunehmen, und dass sich jedes Gelenkstück (12) nach außen erstreckt und über der Oberseite der oberen Kerbe (11) abdeckt.

7. Gelenkbewegungs-Stützschutzvorrichtung nach Anspruch 1, **dadurch gekennzeichnet, dass** eine Vielzahl streifenförmiger Befestigungsgurte (20) für die Befestigungsvorrichtung (2) angenommen wird, ein Ende des Befestigungsgurts (20) und eine Seite des Gelenkschutzkörpers (1) einstückig geformt sind, ein hinteres Ende des Befestigungsgurts (20) mit mindestens einer Schnalle (22) versehen ist, der Gelenkschutzkörper (1) mit einem entsprechenden Durchgangsloch (23) versehen ist, das zu der Schnalle (22) passt; nach dem Umwickeln des Gelenks der Befestigungsgurt (20) an dem Gelenkschutzkörper (1) durch Anbringen der Schnalle (22) an dem Durchgangsloch (23) angebracht wird.

8. Gelenkbewegungs-Stützschutzvorrichtung nach Anspruch 7, **dadurch gekennzeichnet, dass** ein Ende des Befestigungsgurts (20) weiter mit mindestens einem Sägezahnwiderhaken (21) versehen ist, und der Gelenkschutzkörper (1) weiter mit einem entsprechenden Durchgangsloch (23) oder halb durchgehenden Loch (24), das zu dem Sägezahnwiderhaken (21) passt, versehen ist.

9. Gelenkbewegungs-Stützschutzvorrichtung nach Anspruch 1, **dadurch gekennzeichnet, dass** die Befestigungsvorrichtung (2) Befestigungsringe (25) beinhaltet, die an zwei Enden des Gelenkschutzkörpers (1) eingerichtet sind, wobei die Befestigungsringe (25) einstückig mit dem Gelenkschutzkörper (1) geformt sind.

10. Gelenkbewegungs-Stützschutzvorrichtung nach Anspruch 1, **dadurch gekennzeichnet, dass** die Befestigungsvorrichtung (2) ein geschlossener Ring (26) oder ein Spaltring (27) ist.

11. Gelenkbewegungs-Stützschutzvorrichtung nach Anspruch 1, **dadurch gekennzeichnet, dass** die Befestigungsvorrichtung (2) ein elastisches Gewebe ist, das an dem Gelenkschutzkörper (1) befestigt ist, oder dass die Befestigungsvorrichtung (2) ein unabhängiger Befestigungsgurt ist, oder dass die Befestigungsvorrichtung (2) ein Schuh ist.

12. Gelenkbewegungs-Stützschutzvorrichtung nach Anspruch 1, **dadurch gekennzeichnet, dass** der Gelenkschutzkörper (1) an einem Schuh befestigt ist.

## Revendications

1. Dispositif de protection de support de mouvement articulaire, comprenant
un corps de protection d'articulation (1) adapté à une articulation en termes de forme, et un dispositif d'immobilisation (2) en liaison ou contact avec le corps de protection d'articulation (1) et attachant étroitement le corps de protection d'articulation (1) à une articulation d'un corps humain, **caractérisé en ce que** : une section transversale du corps de protection d'articulation (1) est en forme d'arc, une pluralité d'encoches supérieures (11) sont formées dans une partie, proche d'une zone de mouvement articulaire, du corps de protection d'articulation (1) ; les encoches supérieures (11) divisent le corps de protection d'articulation (1) en une pluralité de pièces d'articulation (12) avec certaines parties de liaison du corps de protection d'articulation (1) qui relient des pièces d'articulation (12) adjacentes entre elles, et des pièces d'articulation (12) adjacentes sont capables de tourner autour des pièces de liaison en tant que pivots (13) ; lorsque les pièces d'articulation (12) tournent jusqu'à une position de limitation autour des pivots (13), les pièces d'articulation (12) ne tournent plus étant donné que les pièces d'articulation (12) adjacentes sont en contact les unes avec les autres.

2. Dispositif de protection de support de mouvement articulaire selon la revendication 1, **caractérisé en ce que** des encoches inférieures (14) correspondantes sont en outre formées dans des positions, s'étendant vers le bas depuis un côté fond, de positions correspondantes des encoches supérieures (11), les encoches inférieures (14) correspondent aux encoches supérieures (11) une par une, une profondeur de l'encoche inférieure (14) est inférieure à une profondeur de l'encoche supérieure (11), et une jonction entre l'encoche supérieure (11) et l'encoche inférieure (14) constitue un pivot de rotation (13) entre des pièces d'articulation (12) adjacentes.

3. Dispositif de protection de support de mouvement articulaire selon la revendication 2, **caractérisé en ce qu'**un rapport de la profondeur de l'encoche inférieure (14) sur la profondeur de l'encoche supérieure (11) est de 1:2~1:8.

4. Dispositif de protection de support de mouvement articulaire selon la revendication 2, **caractérisé en ce que** des bords de l'encoche supérieure (11) ou de l'encoche inférieure (14) et de la pièce d'articulation (12) sont arrondis.

5. Dispositif de protection de support de mouvement articulaire selon la revendication 1, **caractérisé en ce qu'**une largeur de fond de l'encoche supérieure (11) est plus grande qu'une largeur de sommet de celle-ci.

6. Dispositif de protection de support de mouvement articulaire selon la revendication 1, **caractérisé en ce que** des hauteurs d'une pluralité des pièces d'articulation (12) augmentent progressivement, et chaque pièce d'articulation (12) s'étend vers l'extérieur et recouvre le sommet de l'encoche supérieure (11).

7. Dispositif de protection de support de mouvement articulaire selon la revendication 1, **caractérisé en ce qu'**une pluralité de sangles d'immobilisation en forme de bande (20) sont adoptées pour le dispositif d'immobilisation (2), une extrémité de la sangle d'immobilisation (20) et un côté du corps de protection d'articulation (1) sont moulés d'un seul tenant, une extrémité de queue de la sangle d'immobilisation (20) est pourvue d'au moins une boucle (22), le corps de protection d'articulation (1) est pourvu d'un trou traversant (23) correspondant adapté à la boucle (22) ; après l'enveloppement de l'articulation, la sangle d'immobilisation (20) est fixée au corps de protection d'articulation (1) en fixant la boucle (22) au trou traversant (23).

8. Dispositif de protection de support de mouvement articulaire selon la revendication 7, **caractérisé en ce qu'**une extrémité de la sangle d'immobilisation (20) est en outre pourvue d'au moins un ardillon en dents de scie (21), et le corps de protection d'articulation (1) est en outre pourvu d'un trou traversant (23) ou trou semi-traversant (24) correspondant adapté à l'ardillon en dents de scie (21).

9. Dispositif de protection de support de mouvement articulaire selon la revendication 1, **caractérisé en ce que** le dispositif d'immobilisation (2) inclut des anneaux d'immobilisation (25) agencés sur deux extrémités du corps de protection d'articulation (1), les anneaux d'immobilisation (25) sont moulés d'un seul tenant avec le corps de protection d'articulation (1).

10. Dispositif de protection de support de mouvement articulaire selon la revendication 1, **caractérisé en ce que** le dispositif d'immobilisation (2) est un anneau fermé (26) ou un anneau fendu (27).

11. Dispositif de protection de support de mouvement articulaire selon la revendication 1, **caractérisé en ce que** le dispositif d'immobilisation (2) est un tissu élastique immobilisé sur le corps de protection d'articulation (1), ou le dispositif d'immobilisation (2) est une sangle d'immobilisation indépendante, ou le dispositif d'immobilisation (2) est une chaussure.

12. Dispositif de protection de support de mouvement articulaire selon la revendication 1, **caractérisé en ce que** le corps de protection d'articulation (1) est immobilisé sur une chaussure.
